(19) Europäisches Patentamt
European Patent Office
Office européen des brevets

(11) Publication number: **0 468 611 A2**

## (12) EUROPEAN PATENT APPLICATION

(21) Application number: **91300561.7**

(22) Date of filing: **24.01.91**

(51) Int. Cl.5: **A61B 5/22**, A61B 5/0436

(30) Priority: **23.07.90 US 556305**

(43) Date of publication of application:
**29.01.92 Bulletin 92/05**

(84) Designated Contracting States:
**DE FR GB IT**

(71) Applicant: **Thornton, William Edgar**
**701 Coward's Creek Road**
**Friendswood, Texas 77546(US)**

(72) Inventor: **Thornton, William Edgar**
**701 Coward's Creek Road**
**Friendswood, Texas 77546(US)**

(74) Representative: **Atkinson, Ralph et al**
**Fitzpatricks Europe House World Trade**
**Centre**
**London E1 9AA(GB)**

(54) **Musculoskeletal activity monitoring system.**

(57) A musculoskeletal activity monitoring system which includes sensors adapted to be affixed to a subject for detecting certain physical activities of the subject and for also detecting the emotional state of the subject. The signals generated by the sensor means are recorded and processed so as to obtain appropriate data for use in orthopedic and metabolic studies and the like.

Fig. 1

EP 0 468 611 A2

Copending United States Patent Applications Serial No. 07/554,421 filed 19th July 1990, Serial No. 07/554,549 filed 19th July 1990, and Serial No. 07/555,307 filed 20th July 1990 (hereinafter referred to as the "Copending Applications") describe and claim various aspects of a cardiovascular monitoring system in which various physical activities of a subject are monitored along with the emotional state of the subject, and certain ambient conditions, to determine what effect, if any, the physical activities and emotional state of the subject have on abnormalities in the subject's EKG.

In such monitoring systems the monitored signals relating to the physical activity and emotional state of the subject are recorded in conjunction with the recording of the EKG signals. It is suggested in the Copending Applications that the signals relating to the physical activities and emotional state of the subject may be recorded together with the EKG signals in a single Holter EKG magnetic tape recorder. In such a case, the latter signals are digitized and multiplexed so that they may be recorded on an unused channel in the Holter recorder. As an alternative, and as also suggested in the Copending Applications, the signals relating to the physical activities and emotional state of the subject may be recorded on a separate recorder.

The use of a separate recorder has certain advantages since it enables the cardiac monitoring system of the Copending Applications to be used in other fields such as orthopedics and metabolic studies. An objective of the present invention is to provide such a monitoring system which is particularly constructed for use in such other fields.

Specifically, an objective of the present invention is to provide a musculoskeletal activity monitor (MSAM) in which data of the type derived from the various sensors described in the Copending Applications is used.

The invention provides a musculoskeletal activity monitor (MSAM) for use in orthopedic and metabolic studies, in which the data from various sensors used to monitor physical activities and the emotional state of a subject are stored and processed in appropriate data reduction units to provide the desired information.

Accordingly, the present invention provides a musculoskeletal activities monitoring system characterised in that it comprises: first sensor means adapted to be affixed to a subject for generating signals related to certain selected physical activities of the subject; second sensor means adapted to be affixed to the subject for generating signals related to the emotional state of the subject; recording means connected to said first and second sensor means for recording the signals generated thereby; and processing means adapted to be connected to said recording means and responsive to the signals recorded thereon for processing data related to the musculoskeletal activities of the subject.

The foregoing and further features of the present invention will be more readily understood from the description of a preferred embodiment, by way of example thereof, and with reference to the accompanying drawings, of which:-

FIGURE 1 is a representation of a subject on which various sensors and other instruments are mounted for carrying out the desired musculoskeletal monitoring funtions;

FIGURES 2A and 2B constitute a further representation of the subject shown in FIGURE 1, and illustrate the manner in which first and second microphones are mounted on the subject for purposes to be explained, FIGURE 2B being a section taken along the line 2B-2B of FIGURE 2A;

FIGURE 3 is a block diagram of a replay/analysis unit which is used to receive data from the recorders carried by the subject of FIGURE 1;

FIGURE 4 is a block diagram of the manner in which vertical acceleration signals of the subject are processed;

FIGURE 5 is a block diagram of a master digital clock which is included in the recorders forming a part of the monitoring system;

FIGURE 6 is a block diagram showing the manner in which signals representing the speech of the subject and others are processed in the system;

FIGURE 7 is a schematic representation of the incidence of speech by the subject and by others monitored by the system;

FIGURE 8 is a series of curves representing the voice pattern of the subject of FIGURE 1 during normal conditions;

FIGURE 9 is a series of curves showing the voice pattern of the subject of FIGURE 1 during emotional conditions;

FIGURE 10 is a block diagram of a circuit which responds to the voice pattern of the subject to determine the emotional state of the subject;

FIGURE 10A is a series of curves useful in explaining the operation of the circuit of FIGURE 10;

FIGURE 11 is a block diagram of a circuit for detecting the incidence of speech from others apart from the subject and also for detecting ambient noise;

FIGURE 12 is a series of curves illustrating the probable ambulatory state of the subject derived from a reading of his or her step rate;

FIGURE 13 is a series of curves for further illustrating the ambulatory state of the subject further enhanced by a measure of the vertical forces generated by the subject;

FIGURE 14 is a series of curves graphically illustrating the results obtained by the monitoring system; and

FIGURE 15-17 are block diagrams of various components required in adapting the augmented Holter monitor (AHM) of the Copending Applications to the musculoskeletal activity monitor (MSAM) of the present invention.

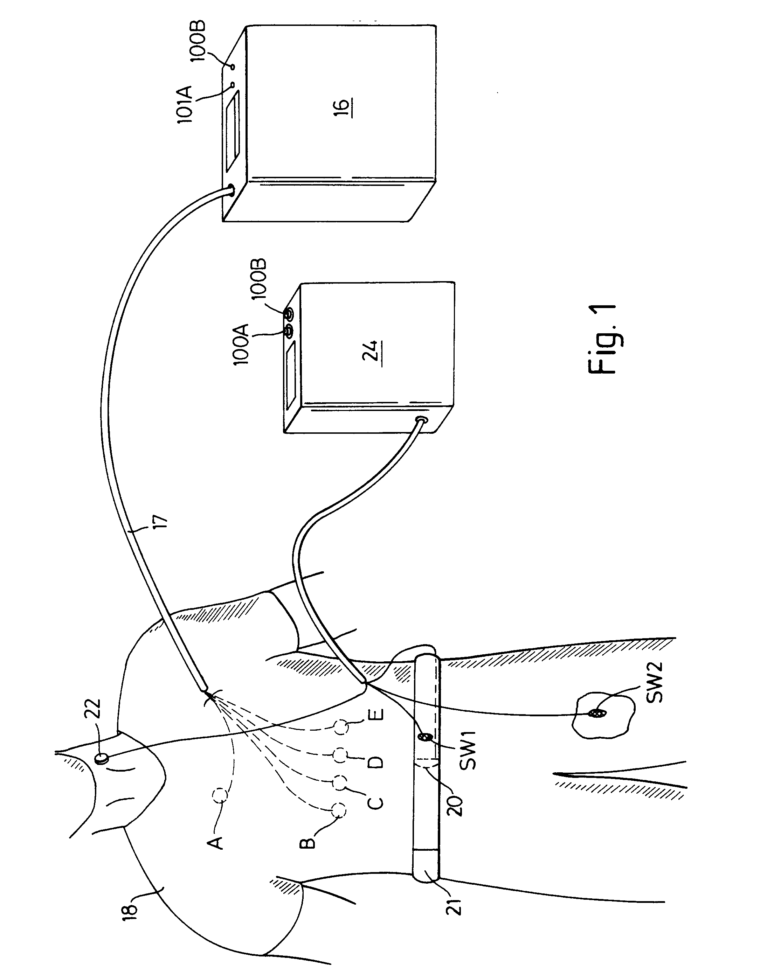

In order for the musculoskeletal activity monitor (MSAM) of the invention to perform its desired monitoring functions, it is necessary for the subject 18 of FIGURE 1 to carry certain sensors, transducers and other equipment. As described in the Copending Applications, the subject 18 may carry an existing miniature EKG Holter magnetic tape recorder 16 in one of his shirt pockets. Usual EKG electrodes A-E are mounted on the subject and connected to the Holter recorder 16 over leads 17. In addition, the heart rate of the subject must be monitored and recorded.

The subject 18 also carries a miniature accelerometer 2 on a belt 21, the accelerometer measuring vertical accelerations (Gz) of the subject at his center of gravity. The accelerations (Gz) may be exactly converted to vertical forces (Fz) by the system in a manner fully described in U.S. Patent 4,830,021, or may be estimated.

Two position sensor switches SW1 and SW2 are also attached to subject 18, one at his waist and the other on his thigh. Switches SW1 and SW2 may be commerically available mercury gravity switches, or other appropriate gravity switches may be used. These switches serve to provide indications of the posture of the subject, specifically whether the subject is standing, sitting or lying down. The operation of the switches SW1 and SW2 is described in some detail in U.S. Patent 4,830,021.

A multiple sensor 22 is mounted on the neck of subject 18. This multiple sensor may include two microphones, as will be described, as well as light and temperature sensors. The light sensor may be a simple photodiode circuit which generates electrical signals indicative of ambient light levels. The temperature sensor may be a thermistor circuit which generates electric signals indicative of ambient temperature. The accelerometer 2, sensor 22, and switches SW1 and SW2 are all connected to a second recorder 27 which may fit into a second shirt pocket of subject 18, or which may be clipped to the Holter recorder 16.

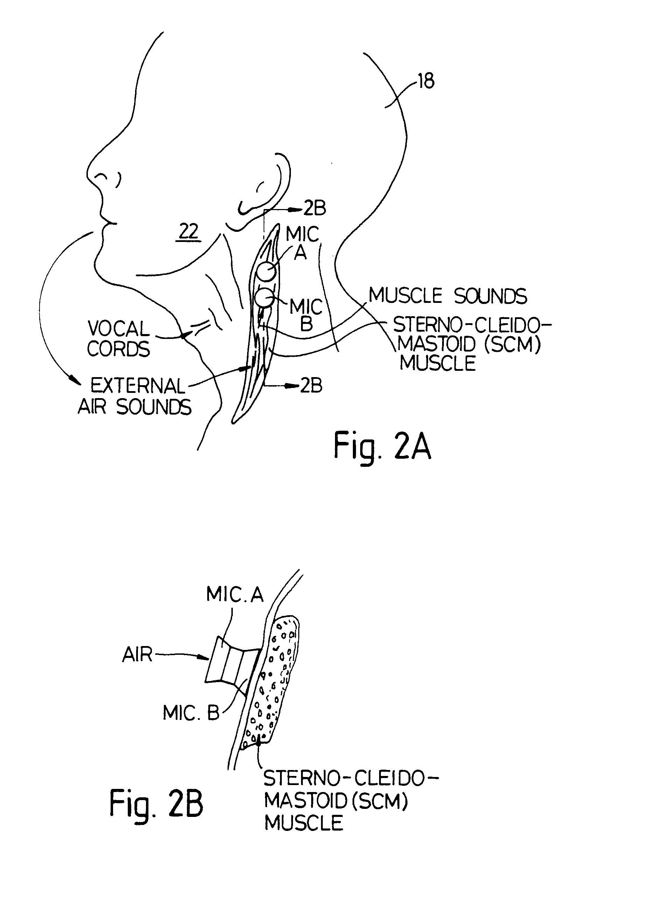

As shown in FIGURES 2A and 2B, composite sensor 22 includes two microphones designated Mic "A" and Mic "B". Microphones Mic "A" and Mic "B" may be sub-miniature microphones of the dynamic, electret or semiconductor type, and preferably have frequency responses in the range of 20-3000 Hz. Microphone Mic "B" is attached to the neck of subject 18 adjacent to the sterno-cleido-mastoid (SCM) muscle above the collar. Microphone Mic "B" registers strong vibrations from the voice of subject 18 (.3-3 KHz); weaker vibrations from external sources including voices (.3-3 KHz); and lower frequency vibrations due to muscle contractions of the subject occurring in REM sleep, for example, when the subject is asleep and dreaming.

Microphone Mic "B" should have a high low frequency response at approximately 10 Hz to repond to the low frequency muscle activity (16 Hz). Although microphones Mic "A" and Mic "B" are shown displaced from one another in FIGURE 2A, microphone Mic "A", is preferably mounted on microphone Mic "B" as shown in FIGURE 2B, and the microphones are acoustically isolated from one another. Microphone Mic "A" serves to register the speech of the subject as transmitted through air, and it also registers other sounds transmitted to it by air.

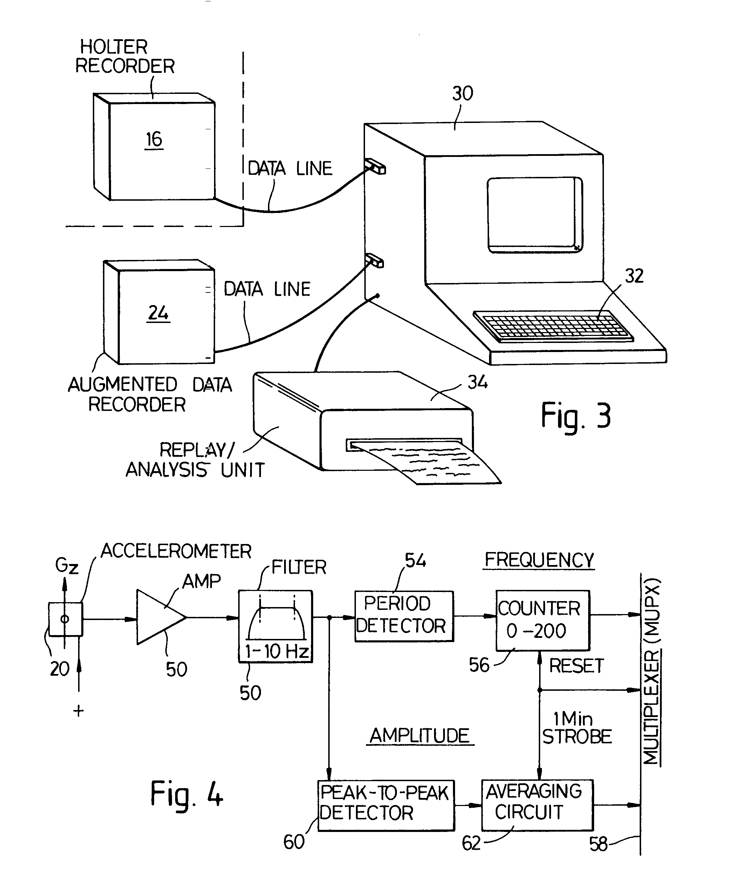

A processing unit 30 is shown in FIGURE 3. Recorder 16 may be connected to unit 30 during replay when an EKG record is to be processed. Recorder 24 of FIGURE 1 is also connected to unit 30 during replay so that the data monitored by the system may be processed by the unit. Unit 30 includes all usual components, including a computer, controls, displays, a keyboard 32 and a printer 24, all of which are needed for digitizing, storing, processing and displaying the data fed into the unit 30.

Correlation and analysis of the data from recorder 24 by unit 30 serves to yield substantial amounts of information on the physical activity and emotional state of subject 18 of FIGURE 1, and of the environment surrounding the subject. An important feature of the processing is that no attempt is made for an absolute analysis, but rather to provide a statisical statement using estimated or known correlation coefficients with respect to the various parameters. In this manner, the probability of the occurrence of any particular event is provided to the physician without the need for excessively large and/or complicated equipment, which still cannot provide absolute certainty.

The vertical acceleration (Gz) outputs of accelerometer 20 of FIGURE 1, which represents the locomotor activity of the subject, has a characteristic waveform which varies synchronously both in frequency and

3

amplitude with the step frequency of the subject. Accordingly, it is important that these two characteristics of the waveform be recorded in recorder 24. The system response of the body is such that a sample rate of one per minute, that is, the number of steps and mean force, is adequate. A circuit for achieving the foregoing may be incorporated into recorder 24. Such a circuit is shown in the block diagram of FIGURE 4.

In FIGURE 4, accelerometer 20 is connected to an amplifier 50 which, in turn, is connected through a bandpass filter 52 to a period detector 54. The period detector 54 is connected to a counter 56 for detecting the frequency of the waveform. The output of counter 56 is connected to a bus 58 which carries the signals from the counter to an appropriate multiplexer (MUPX). Filter 52 is also connected to a peak-to-peak detector 60 for detecting the amplitude of the waveform. The output of peak-to-peak detector 60 is connected to an averaging circuit 62 whose output is also connected to bus 58. Counter 56 and integrator circuit 62 are each reset by an appropriate one minute strobe, as shown. The multiplexer MUPX causes the data from the circuit of FIGURE 4 to be recorded in recorder 24, multiplexed with other data from the sensors of FIGURE 1. All of the data is preferably recorded in digital form.

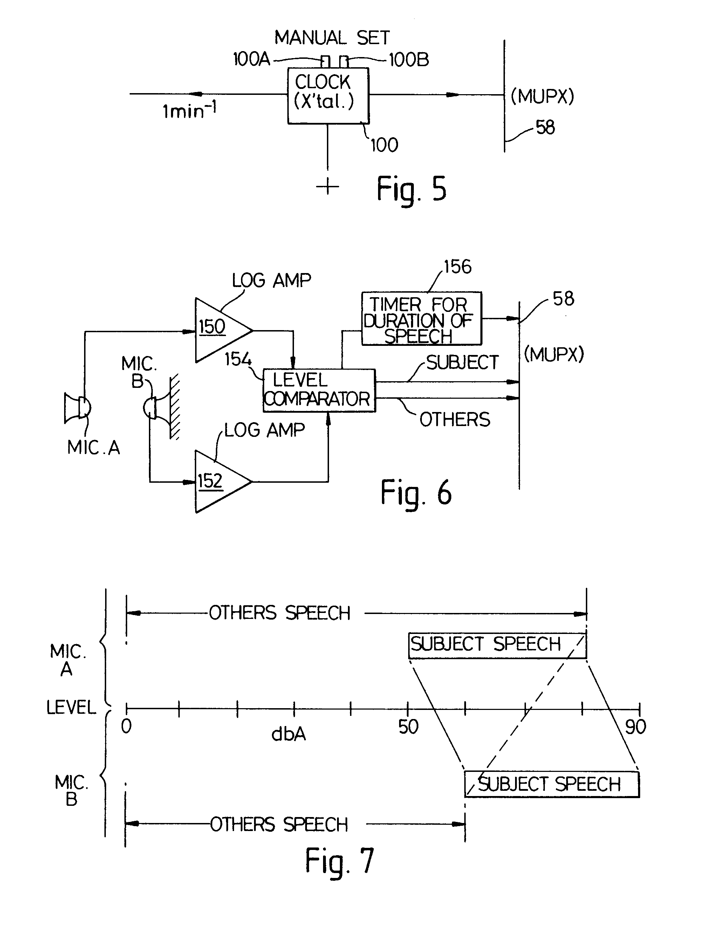

A crystal time clock 100, as shown in FIGURE 5, is included in recorder 24. The time clock may include the usual manual set controls 100A, 100B which are also shown in FIGURE 1. Discrete twenty-four hour time signals and a 1 min-1 code is generated by the clock 100. The discrete time signals are introduced to bus 58 which carries the signals to the multiplexer MUPX to be recorded in digital form in recorder 24 mutliplexed with the other digital data. A similar time clock may be included in recorder 16 (FIGURE 1) with manual time set controls 101A and 101B. such a clock would produce only time signals in a single unit recorder.

The outputs from microphones Mic "A" and Mic "B" of FIGURE 2A and 2B are amplified by respective log amplifiers 150 and 152 in the circuit of FIGURE 6, which circuit is included in recorder 24. The amplified outputs from the log amplifiers are compared in a level comparator 154 for amplitude differences. The comparator 154 provides output signals which distinguish the subject's speech from the speech of others, and such signals are applied to bus 58 to be carried to multiplexer MUPX and to be recorded in recorder 24 in digital form. The circuit of FIGURE 6 also includes a timer 156 which provides time signals for timing the duration of speech components of the subject and of others. The speech of the subject and the speech of others may be distinguished because of amplitude differences, as shown in the representation of FIGURE 7.

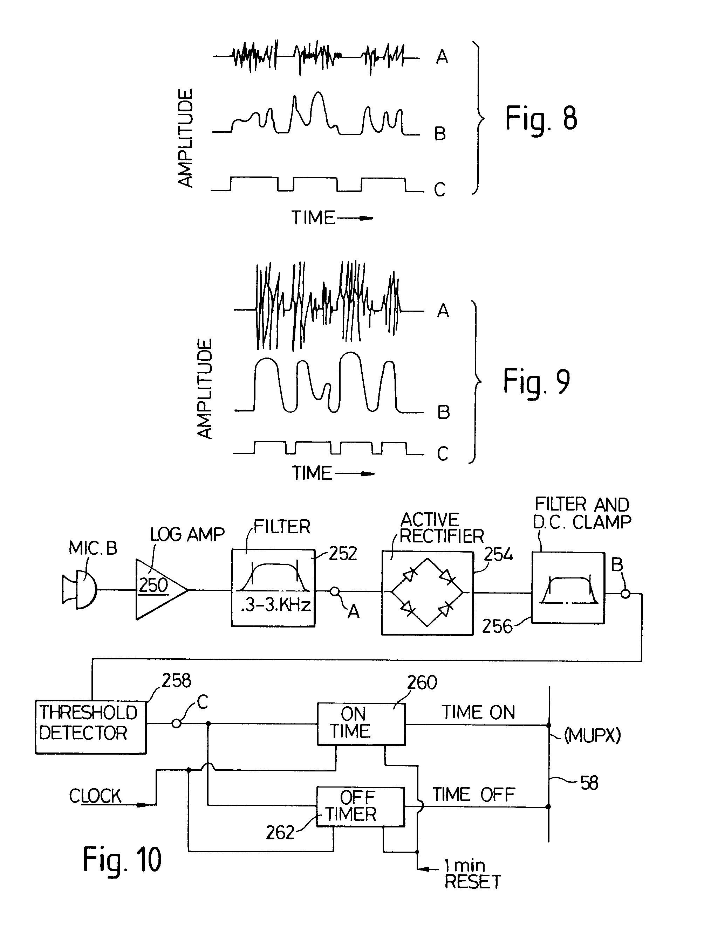

The normal voice of the subject 18, when not under emotional stress, is represented by curves A, B, and C in FIGURE 8; and the voice of the subject when under emotional stress is represented by the curves of FIGURE 9. The speech of the subject and its emotional content is detected by the duty cycle of the sound envelopes, and this is achieved by comparing the ratios of the on and off times of the speech of the subject. That is, as the subject becomes emotional there will be less space between the words and the words will be shorter. It is also desirable to include an amplitude detector, since a raised voice is a usual concomitant of emotion.

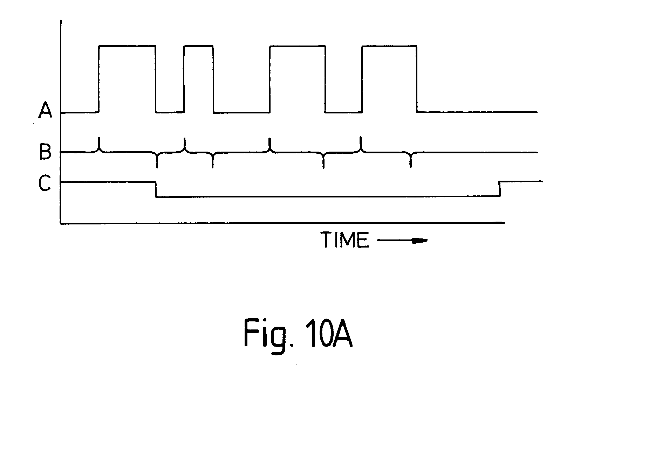

A circuit for detecting the emotional content of the speech of the subject is included in recorder 24, and a typical circuit is shown in block diagram in FIGURE 10. In FIGURE 10, the microphone Mic "B" is connected to a log amplifier 50 which, in turn, is connected through a bandpass filter 252 to a precision active rectifier 254. The output of rectifier 254 to a threshold detector 258. The threshold detector is connected to the "on" input of a timer 260 which measures the "on and "off" time of the speech.

The "off" time must be limited after an "on" cycle, otherwise it will count long periods, for example, between sentences.

Such limitation is achieved by a fixed length delay circuit 262 and a "not" gate 264. The output of the "not" gate is connected to the "off" input of timer 260. The output of threshold detector 258 is also applied to a differentiating circuit C1, R1 which is connected to delay circuit 262 through a diode D1.

Curves A, B and C of FIGURE 10A represent the waveform of the various signals in the circuit of FIGURE 10. Curve A is the on/off signal, which is differentiated by the differentiator C1, R1 to generate a negative-going trigger (curve B) each time a signal A goes negative. Trigger signal B triggers delay circuit 262 at time intervals longer than the usual delay between words. Delay circuit 262 is reset by each negative trigger B, and when it is not reset, its output C is high.

Timer 260 is turned on whenever signal A is low, and it is turned off whenever signal A is low. Should signal A remain low after a predetermined time interval established by delay circuit 262, it is turned off by trigger C going high while signal A is low by virtue of "not" gate 264.

As shown, the normal voice and emotional voice of the subject appears at the output of filter 252, as represented by the curve A in each of the diagrams of FIGURES 8 and 9. The rectified waveforms B of FIGURES 8 and 9 appears at the output of the filter and clamp circuit 256 in FIGURE 10, and that output is transformed into a series of pulses by threshold detector 258, the pulses being shown by curves C in FIGURES 8 and 9. The closer the pulses in the curves C are together, and the shorter the pulses, the more emotional is the speech of subject 18. Pulses from the threshold detector 258 are applied to the timers 260 and 262, and reading of the on time and off time of each of the pulses is fed to the multiplexer over bus 58 to be recorded in recorder 24.

The circuit of FIGURE 10 also includes a real detector 257 and an averaging circuit 258, which serve as an amplitude detector, for the reasons stated above.

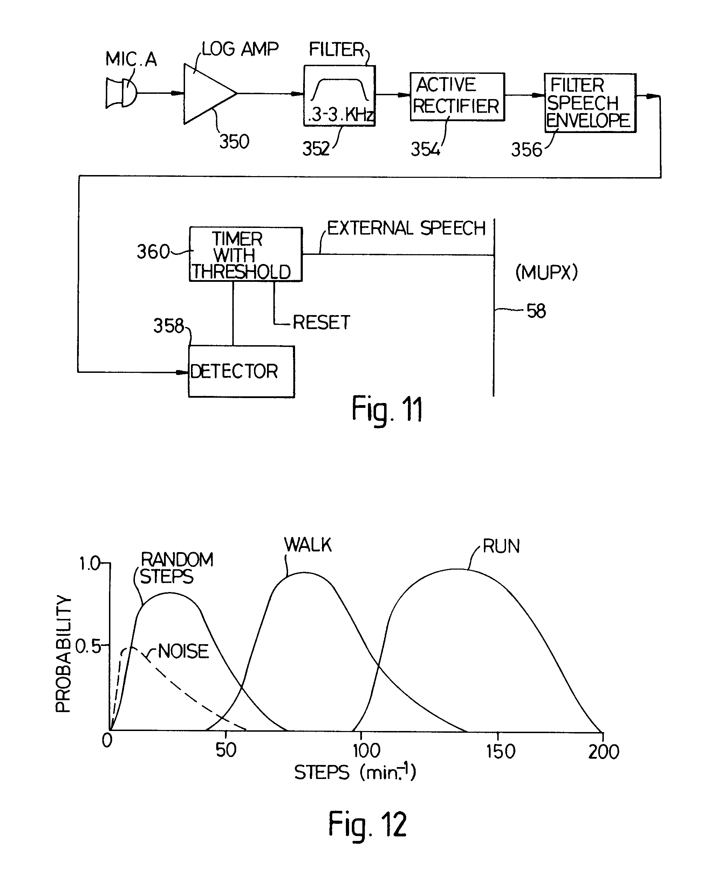

As mentioned above, the speech of others and external noise is detected by microphone Mic "A", and is processed by the circuit of FIGURE 11. Microphone Mic "A" is connected to a log amplifier 350 which, in turn, is connected through a bandpass filter 352 to an active rectifier 354. The output of the active rectifier is passed through a filter 356 whose output, in turn, is passed to a detector 358. Output of detector 358 is connected to a timer 36 who se output, in turn, is fed to the multiplexer MUPX over bus 58.

The circuit of FIGURE 11 provides an output representative of the presence of the speech. Noise is determined by the presence of a continuous background which results in an output from microphone Mic "A" which is of greater amplitude than the output of microphone Mic "B".

As mentioned above, the multiple sensor 22 of Figure 1 which is mounted on the neck of subject 18 also contains a simple photodiode which is encoded into several light levels so that signals representing ambient light levels may be applied to bus 58 and transmitted to multiplexer MUPX. As also mentioned, temperature readings are provided by including a thermistor in sensor 22 and by applying its output to bus 58. The posture of the subject is also detected by applying signals from the posture sensors SW1 and SW2 of FIGURE 1 to bus 58, and utilizing the signals in the manner described in U.S. Patent 4,830,021.

In the foregoing manner, all of the augmented data from sensors 20, SW1, SW2 and 22 in FIGURE 1 is multiplexed and recorded in digital form on recorder 24. Accordingly, the data collected by the system described above includes physical activity which requires increased heart work. The largest load in the usual subject is walking-jogging-running (locomotor activity). The monitoring of such activity is described in U.S. Patent 4,830,021 which discloses a system by which such activity may be accurately detected and recorded.

In addition to physical activity, the monitoring system of the invention serves to detect and record emotional events. These emotional events are detected and recorded in the system of the invention directly, rather than relying on questionable and variable data such as galvanic skin response, as is sometimes used in the prior art. Two events known to be frequently associated with emotional stress are recorded, as described, and these comprise the presence of subjects speech and conversation, as well as dream states. The voice patterns are analyzed for emotional indicators.

Other conditions known either to effect cardiac activity or which may define cardiac activities are also recorded, and these include subject posture, that is lying, sitting, standing; as well as ambient conditions such as air temperature and ambient light level.

Correlation of the data referred to above yields a substantial amount of information on the subject's activity and environment. As mentioned above, an important feature of the analysis is that it does not attempt absolute determination but rather provides a statistical statement, using estimated or known correlation coefficients from the various inputs. That is, the probability of an event will be provided to the physician. Traditionally, data has been more or less continuously recorded on tape in cardiovascular monitoring systems and processed on reply. However, there are too many sources in the present system to render such an approach feasible. While processing portions of the date before recording and recording others is possible, pre-processing and digital recording represent the most practical approach.

The use of a separate recorder 24 enables it to be used in fields such as orthopedics and metabolic studies, which forms the subject matter of the present invention. It is desirable to include circuits in the recorder 24 which would be unused in one or the other of the applications, for example heart rate for metabolic, force for orthopedics, and so on.

An important feature of the system of the invention in one of its aspects is its ability to correlate, analyze and process data from the various sources. For example, the capability of processing and cross-correlating data from six sources (time, locomotor activity, sound, light, temperature and posture) permits one hundred forty-nine combinatmons, even though not all may be useful. Moreover, since multiple parameters may be extracted from each source, the process is generic rather than comrehensive.

Another important feature of the system of the invention is the reductiion of the data to be the minimum required by the user. This reduction of data will now be described for each of the parameters.

A. STEP RATE

Step rate is arbitrarily broken into several categories.

| | |
|---|---|
| 5-50 steps/minute | random steps |
| 50-120 steps/minute | walking |
| 50-80 steps/minute | walking slowly |
| 80-120 steps/minute | walking quickly |
| 120-200 steps/minute | running |
| 120-150 steps/minute | runing slowly |
| 150-200 steps/minute | running quickly |

Taken alone, the above parameters have varying probabilities which are shown in the curves of FIGURE 12. This data forms part of the analysis program.

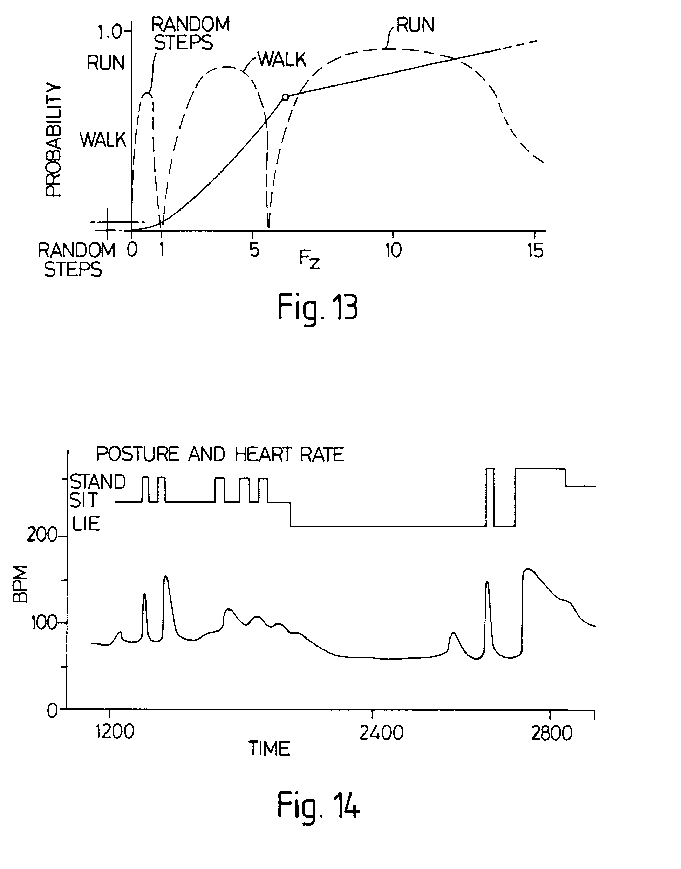

To increase the reliability of the system, vertical force (Fz) should also be used. The monitoring of such force is described in detail in U.S. Patent 4,830,021. The characteristic of the vertical force (Fz) is shown with a probability curve in FIGURE 13.

By combining the probabilities from the step rate and Fz parameters much higher reliability can be obtained especially in the transition zones between the step-walk-run states. For example, if step rate were used alone, there would be no detection of jumping by the subject so that an anomaly could arise. However, jumping would be detected by the resulting large Fz occurring at low step rates.

B. POSTURE

The posture signal can provide information in its own right as well as adding reliability to other signals. For example, only in the standing position can one engage in normal locomotor activity. Sleep is more likely when lying but would almost never occur standing.

C. TIME

Time is an imperative signal for identifying and coordinating activities, but it can also be a valuable adjunct signal in analysis. For example, the probability of sleep is much higher in hours just prior and following midnight, while locomotor activity is much lower.

It should be noted that if the subject reliably reports events and time, this additional data could be manually entered into the computer. For example, if the patient reliably states he went to bed at 2230 hours and got up at 0645 hours, this information can be manually entered and used in correlation.

D. LIGHT

Signals representing light are indicative of the environment, especially when used with other data. For example, above a certain light level and time of day, there is a high probabilit} that the subject is in sunlight, whereas an indication of total darkness suggests the likelihood that the subject is asleep.

E. TEMPERATURE

This is a useful signal in its own right for its affects heart rate but it may be an indicator of indoors versus outdoors.

F. VOICE/SOUND

The discrimination between external speech, subject speech and subject speech with emotion has been described above. Conversation has certain probabilities when the subject and external speech are present.

All of the foregoing functions are processed by the computer which first stores the data, as described above, and then indexes the data for ultimate analyses. The computer could also perform individual characterizations and normalizations of data, for example, to establish averages or totals for data such as 10,4000 steps/day at 130 steps/minute; or to establish normal subject voice levels and speech duty cycles such that individual deviations become more significant.

When desired a complete graphic or other record may be made available by the computer, as showin in FIGURE 14.

As described and claimed in the Copending Applications, the data obtained from the subject by the various sensors may be integrated with a Holter monitoring system to provide an augmented Holter monitor (AHM) by which the augmented data is used to demonstrate its probable effects on the cardiovascular activity of the subject. In accordance with the present invention, the data derived from the various sensors may be used alone to constitute a Musculoskeletal Activity Monitor (MSAM).

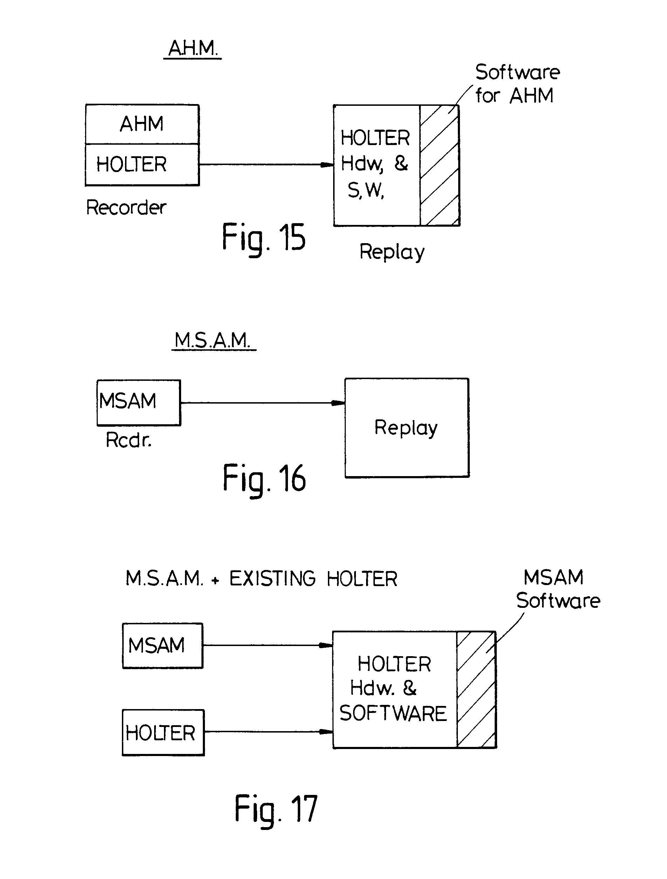

Insofar as the AHM of the present invention is concerned, the system incorporates the data from the various sensors, as stored and processed in the system, into existing designs and with modified software and data reduction units provided to accommodate the data and programs. Such a system is represented generally by the block diagram of FIGURE 15.

The MSAM of the present invention records the data alone and has its own replay unit. Although the MSAM of the present invention could be used to augment the Holter monitor as described in the Copending Applications, its chief utility is in orthopedic and metabolic studies.

The MSAM system of the invention is shown generally by the block diagram of FIGURE 61; and the MSAM system of the invention integrated with an existing Holter monitor, as described in the Copending Applications, is shown generally in the block diagram of FIGURE 17.

Special features of the MSAM of the invention are shown in the following table:

| QUANTITY | AHM | ORTHOPEDIC | METABOLIC |
|---|---|---|---|
| Time | X | X | X |
| Step Rate | X | X | X |
| Foot/Gnd. Force | X | X | X |
| Speech/Sound | X | – | – |
| Heart Rate | X | X | X |
| Temperature | X | X | X |
| Light | X | X | X |
| Posture | X | X | X |
| Rem Sleep | X | – | – |
| Upper Body Activity | X | X | X |

For orthopedic/metabolic applications a different software replay package is required than that required in the cardiovascular monitoring system of the Copending Applications. However, it will operate on the same principles of cross-correlation of data but will give different results.

The invention provides, therefore, an improved monitoring system which monitors physical activities and the emotional state of a subject to obtain data relating to the subject's musculoskeletal activities.

It will be appreciated that while a particular embodiment of the invention has been shown and described, moficiations may be made. It is intended in the claims to cover all modifications which come within the true spirit and scope of the invention.

## Claims

1. A musculoskeletal activities monitoring system characterised in that it comprises:

first sensor means adapted to be affixed to a subject for generating signals related to certain selected physical activities of the subject; second sensor means adapted to be affixed to the subject for generating signals related to the emotional state of the subject; recording means connected to said first and second sensor means for recording the signals generated thereby; and processing means adapted to be connected to said recording means and responsive to the signals recorded thereon for processing data related to the musculoskeletal activities of the subject.

2. The monitoring system as claimed in Claim 1, characterised in that it includes third sensor means adapted to be affixed to the subject for generating signals related to selected ambient conditions, and means connecting said third sensor means to said recording means to enable signals from said third sensor means to be recorded thereby for use by said processing means.

3. The monitoring system as claimed in Claim 1, characterised in that it includes a clock circuit included in said recording means for generating time signals to be recorded in conjunction with the signals from said first and second sensor means.

7

4.  The monitoring system as claimed in Claim 1, characterised in that it includes fourth sensor means adapted to be affixed to the subject for generating signals related to the posture of the subject, and means connecting said fourth sensor means for said recording means to enable signals generated thereby to be recorded by said recording means for use by said signal processing means.

5.  The monitoring system as claimed in Claim 1, characterised in that it includes fifth sensor means for generating signals related to muscle contractions of the subjection, and means connecting said fifth sensor means to said recording means for enabling said recording means to record the signals generated thereby for use by said processing means.

Fig. 1

Fig. 2A

Fig. 2B

Fig. 3

HOLTER RECORDER — 16

DATA LINE — 30

32

AUGMENTED DATA RECORDER — 24

DATA LINE

REPLAY/ANALYSIS UNIT — 34

Fig. 4

ACCELEROMETER

$G_z$

AMP — 50

FILTER — 50 (1-10 Hz)

PERIOD DETECTOR — 54

FREQUENCY

COUNTER 0-200 — 56

RESET

1 Min STROBE

AMPLITUDE

PEAK-TO-PEAK DETECTOR — 60

AVERAGING CIRCUIT — 62

MULTIPLEXER (MUPX) — 58

20 +

11

MANUAL SET

100A — 100B

CLOCK
(X'tal.)

1min⁻¹

(MUPX)

58

100

+

## Fig. 5

LOG AMP

156

150

TIMER FOR
DURATION OF
SPEECH

58

MIC.
B

154

LEVEL
COMPARATOR

SUBJECT

(MUPX)

MIC.A

LOG AMP

OTHERS

152

## Fig. 6

OTHERS SPEECH

MIC.
A

SUBJECT SPEECH

LEVEL

0        dbA        50        90

MIC.
B

SUBJECT SPEECH

OTHERS SPEECH

## Fig. 7

Fig. 8

Fig. 9

Fig. 10

Fig. 10A

Fig. 11

Fig. 12

Fig. 13

Fig. 14

A.H.M.

Software
for AHM

AHM

HOLTER

Recorder

HOLTER
Hdw, &
S,W,

Fig. 15

Replay

M.S.A.M.

MSAM

Rcdr.

Replay

Fig. 16

M.S.A.M. + EXISTING HOLTER

MSAM
Software

MSAM

HOLTER

HOLTER
Hdw. &
SOFTWARE

Fig. 17